# EUROPEAN PATENT APPLICATION

(11) **EP 2 309 003 A1**
(43) Date of publication of application: **13.04.2011**
(21) Application number: 09382171.8
(22) Date of filing: 18.09.2009
(51) Int. Cl.: C12Q 1/68

(54) **Method for the diagnosis of limbal stem cell deficiency**

(71) Applicant: Bioftalmik, S.L., 48160 Derio (Vizcaya) (ES)
(72) Inventor: García Jimenez, Iker, 48160 Derio (Vizcaya) (ES); Gonzalez Fernandez, Nerea, 48160 Derio (Vizcaya) (ES); Soria Esponera, Javier, 48160 Derio (Vizcaya) (ES); Acera Osa, Arantxa, 48160 Derio (Vizcaya) (ES); Suárez Cortés, Tatiana, Parque Tecnológ, Derio (Vizcaya) (ES)
(74) Representative: ABG Patentes, S.L.

(57) **Abstract**

The invention relates to a method for the diagnosis of limbal stem cell deficiency (LSCD) in a subject, based on detecting or quantifying the expression of the *MUC5AC* gene in a cornea sample from said subject.

SEQUENCE LISTING

<110>  BIOFTALMIK, S.L.

<120>  METHOD FOR THE DIAGNOSIS OF LIMBAL STEM CELL DEFICIENCY

<130>  P4708EP00

<160>  2

<170>  PatentIn version 3.5

<210>  1
<211>  19
<212>  DNA
<213>  Artificial Sequence

<220>
<223>  (Forward) oligonucleotide primer MUC5AC-RT-F2 for amplifying
       a fragment of the MUC5AC gene

<400>  1
cctgcaagcc tccaggtag                                                    19


<210>  2
<211>  18
<212>  DNA
<213>  Artificial Sequence

<220>
<223>  (Reverse) oligonucleotide primer MUC5AC-RT-R2 for amplifying
       a fragment of the MUC5AC gene

<400>  2
ctgctccact ggctttgg                                                     18

## Description

### Field of the Invention

The invention is comprised within the field of the area of diagnosis of diseases; specifically, in the development of a specific, sensitive and reliable method for the diagnosis of limbal stem cell deficiency in a subject based on the expression of the *MUC5AC* gene in the cornea.

### Background of the Invention

Limbal stem cell deficiency is a clinical entity occurring due to the destruction of limbal stem cells. Said stem cells are located in an area of transition between the columnar conjunctival epithelium and the stratified squamous corneal epithelium called "limbus". Multiple functions are developed in the limbus, such as the nutrition of the peripheral cornea, corneal healing and sensitivity responses.

These cells are believed to be responsible for the regenerative function allowing the maintenance of the corneal epithelium and for the barrier function against the migration of conjunctival cells on the cornea [Dua et al., Surv Ophthalmol (2000); 44:415-425]. The loss of said functions is known as limbal deficiency or limbal stem cell deficiency (LSCD), and can be the consequence of the direct destruction of said cell population or of its stromal microenvironment. Histopathologically, LSCD is characterized by the existence of conjunctivalization with the presence of goblet cells on the cornea, vascularization, destruction of the corneal basement membrane and chronic inflammation [Puangsricharern et al., Ophthalmology (1995); 102:1476-1485). The treatment of total LSCD requires transplanting a sufficient amount of limbal stem cells to achieve corneal reepithelialization with cells with the suitable phenotype.

The loss or dysfunction of said stem cells of the corneal epithelium in a sufficient number translates into the incapacity to maintain the dynamic equilibrium of the corneal epithelium and into the onset of the pathological condition (LSCD). When this occurs, and to prevent an epithelial defect, there is an invasion of the conjunctival epithelium in the cornea (a process known as "conjunctivalization") which, in the absence of blood vessels, adopts a phenotype similar to the corneal phenotype although it never manages to transdifferentiate completely; this process is normally accompanied by subepithelial vascularization (with chronicity it constitutes the fibrovascular tissue known as "pannus" and the corneal transparency is altered), with persistent epithelial defects and stromal healing. In more severe cases persistent epithelial defects, calcifications, stromal ulcers and even perforations occur. Clinically, there is a loss of transparency of the cornea which, if it affects its central area, causes a decrease in visual acuity. Other symptoms with which it is associated include photophobia, lacrimation, blepharospasm, recurrent episodes of pain and chronic inflammation with reddening and edema [Annals d'Oftalmologia (2001); 9(3):149-151].

In Spain the incidence of limbus deficiency is estimated at about 5/1000 inhabitants/year. The clinical diagnosis of LSCD must be intuited with the presence of an irregular epithelium, without shine, which stains anomalously with fluorescein (since the conjunctival epithelium is more permeable than the corneal epithelium). The presence of blood vessels which normally accompany the conjunctival epithelium makes LSCD more evident. Generally, the conjunctivalization process can theoretically be detected by the presence of goblet cells in an impression cytology of the corneal epithelium; therefore the definitive diagnosis of LSCD is currently based on a histological method for confirming the existence of an epithelium with goblet cells which are typical of the conjunctiva only and which migrate from the conjunctiva to the cornea in patients with LSCD. In fact, LSCD is currently diagnosed by means of impression cytology using PAS-hematoxylin staining and/or immunocytochemistry to detect the MUC5AC protein by means of specific antibodies in the cornea. Impression cytology is a non-invasive method for obtaining histological information, which has allowed its clinical application in the diagnosis of ocular surface pathologies. These methods are less sensitive and specific due to the material of the filters (cellulose acetate), on which the epithelium cells are collected, being stained with the PAS stain and leading to false positives, or to the preparation not being well stained and to not being able to discriminate between a goblet cell or depositions of the actual stain.

Tseng *et al*. [Tseng SCG et al. Am. J. Ophtalmol. (1997), 124:825-35] retrospectively studied 134 clinically suspected cases of LSCD and said suspicion could not be confirmed by means of impression cytology in 40 cases (30%) (only squamous metaplasia was detected in these cases); they were cases in which a sufficient loss of stem cells to cause a conjunctivalization of the cornea probably did not occur although there were clinical signs such as vascularization, fibrosis and epithelial defects which led to presupposing it.

Pauklin *et al*. [Pauklin et al. "Limbal stem cell deficiency after chemical burns: Investigations on the epithelial phenotype and inflammation status", Ophtalmologe, (2009) Jan 24] analyze the expression of the epithelial strain markers K3, K19 and MUC5AC and of the inflammatory markers IL-1beta, ICAM-1 and VEGF by means of Western Blot and/or real-time polymerase chain reaction in the cornea and conjunctiva reaching the conclusion that the expression of K9 and MUC5AC in normal (healthy) corneal tissue was lower than the expression of said markers in normal conjunctiva.

It is therefore necessary to develop a method for the diagnosis of LSCD which overcomes the mentioned drawbacks; it would be particularly desirable for said method to have a high sensitivity and/or specificity and to generate less false negatives than the methods usually used in the diagnosis of LSCD.

### Summary of the Invention

The inventors have now found that the detection of the *MUC5AC* gene in a cornea sample from a subject is indicative of said subject suffering from LSCD. A number of assays conducted by the inventors have clearly shown that the detection of the *MUC5AC* gene by means of reverse transcription (RT) and real-time polymerase chain reaction (qPCR) in a cornea sample from a subject, using a suitable pair of oligonucleotide primers, such as the one formed by the oligonucleotide primer MUC5AC-RT-F2 comprising the nucleotide sequence shown in SEQ ID NO: 1 and the oligonucleotide primer MUC5AC-RT-R2 comprising the nucleotide sequence shown in SEQ ID NO: 2, allows diagnosing LSCD in a sensitive and specific manner with a higher correlation between the clinical diagnosis and the impression cytology analyzed by RT-qPCR than between the clinical diagnosis and the conventional impression cytology stained with PAS-hematoxylin (Example 1).

Therefore, in one aspect, the invention relates to an *in vitro* method for diagnosing LSCD in a subject based on detecting the expression of the *MUC5AC* gene in a cornea sample from said subject; the expression of said *MUC5AC* gene in the cornea sample is indicative of the subject suffering from LSCD.

In another aspect, the invention relates to an *in vitro* method for diagnosing LSCD in a subject based on an increased expression of the *MUC5AC* gene in a cornea sample from said subject with respect to the expression level of said gene in a reference sample. In a particular embodiment, the expression of the *MUC5AC* gene is quantified by means of RT-qPCR using a pair of oligonucleotide primers formed by the oligonucleotide primer MUC5AC-RT-F2 comprising the nucleotide sequence shown in SEQ ID NO: 1 and the oligonucleotide primer MUC5AC-RT-R2 comprising the nucleotide sequence shown in SEQ ID NO: 2, under suitable conditions.

In another aspect, the invention relates to an oligonucleotide selected from an oligonucleotide comprising the nucleotide sequence shown in SEQ ID NO: 1, an oligonucleotide comprising the nucleotide sequence shown in SEQ ID NO: 2 and combinations of both oligonucleotides.

In another aspect, the invention relates to a kit comprising at least one oligonucleotide selected from an oligonucleotide comprising the nucleotide sequence shown in SEQ ID NO: 1, an oligonucleotide comprising the nucleotide sequence shown in SEQ ID NO: 2 and combinations thereof; or, alternatively, the pair of oligonucleotide primers formed by the oligonucleotide primer MUC5AC-RT-F2 comprising the nucleotide sequence shown in SEQ ID NO: 1 and the oligonucleotide primer MUC5AC-RT-R2 comprising the nucleotide sequence shown in SEQ ID NO: 2; said kit can be used for the diagnosis of LSCD in a subject, therefore the use of said kit for diagnosing LSCD is an additional aspect of the invention.

In another aspect, the invention relates to a method for evaluating if a patient with a corneal pathology is a suitable candidate for corneal transplantation or for a keratoplasty.

### Brief Description of the Drawings

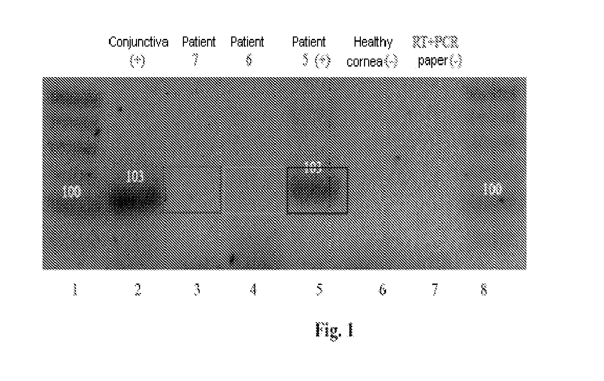

Figure 1 is a photograph of an electrophoresis gel showing the results of the analysis of cornea samples from patients 5, 6 and 7 by means of RT-PCR and subsequent electrophoresis in 2.5% agarose gel and developing with ethidium bromide; lane 5 shows a band corresponding to the specific 103 base pair (bp) fragment of the mRNA of the *MUC5AC* gene amplified with the pair of oligonucleotide primers formed by the oligonucleotides MUC5AC-RT-F2 (SEQ ID NO: 1) and MUCSAC-RT-R2 (SEQ I D NO: 2), representative of the expression of the *MUC5AC* gene in the cornea of patient 5. Lanes 1 and 8: Molecular weight markers; Lane 2: Positive reference sample of healthy conjunctiva; Lanes 3, 4 and 5: Cornea samples from patients 7, 6 and 5; Lane 6: Negative reference sample of healthy cornea; and Lane 7: Negative control of filter without biological sample.

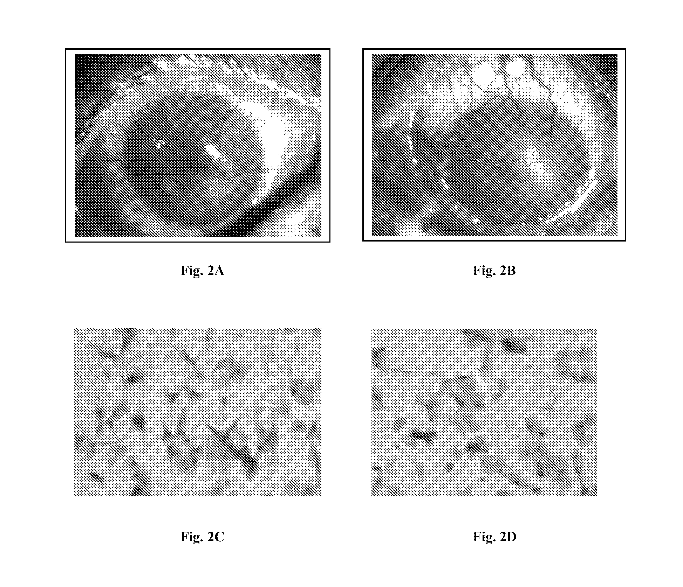

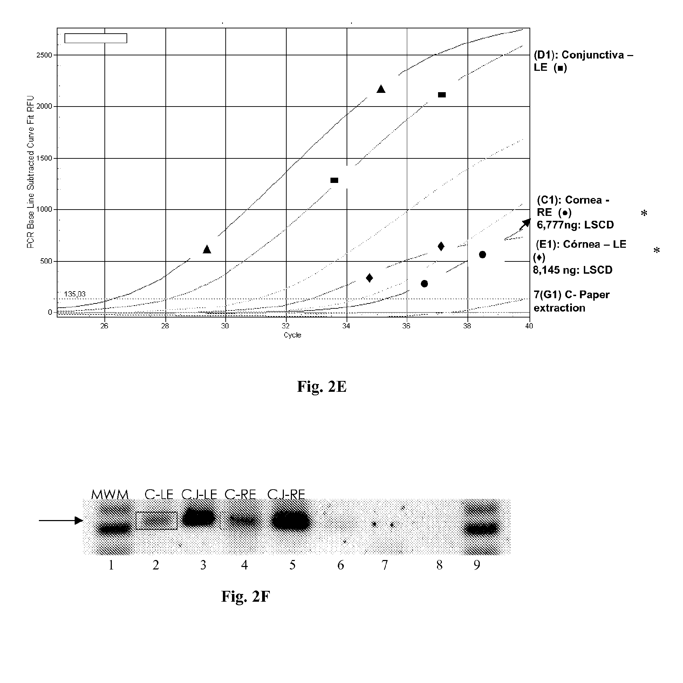

Figure 2 comprises a set of photographs showing a diagnosis of LSCD by means of a slit lamp examination (Figures 2A and 2B), impression cytology and PAS-hematoxylin staining (Figures 2C and 2D) and by means of RT-qPCR (Figures 2E and 2F) of patient 0216. Figure 2C is a microphotograph of corneal cells from the left eye (LE) of patient 0216 at a magnification of 20 (20X), wherein goblet cells can be seen. Figure 2D is a microphotograph of corneal cells from the right eye (RE) of the same patient (patient 0216) at 20X, wherein the presence of goblet cells can also be seen. Figure 2E is a graph showing the amplification curves obtained by means of RT-qPCR of the specific fragment of the *MUC5AC* gene amplified with the pair of oligonucleotide primers formed by the oligonucleotides MUC5AC-RT-F2 (SEQ ID NO: 1) and MUC5AC-RT-R2 (SEQ ID NO: 2) and using a specific fluorescent probe [sequence 5'-CTGGCTGC-3', labeled with 6-FAM at the 5' end] in cornea and conjunctiva samples from the left eye (LE) and right eye (RE) of patient 0216; LSCD: Limbal stem cell deficiency. Figure 2F is a photograph of an electrophoresis gel showing the results of the analysis of cornea samples from the left and right eyes of patient 0216 by means of RT-PCR and subsequent electrophoresis in 2.5% agarose gel and developing with ethidium bromide; lanes 2 and 4 show respective bands corresponding to the specific 103 bp fragment of the mRNA of the *MUC5AC* gene amplified with the pair of oligonucleotide primers formed by the oligonucleotides MUC5AC-RT-F2 (SEQ ID NO: 1) and MUC5AC-RT-R2 (SEQ ID NO: 2), representative of the expression of the *MUC5AC* gene in the corneas of both eyes of patient 0216. Lanes 1 and 9: Molecular weight markers (MWM); Lane 2 (C-LE): Cornea sample from the left eye of patient 0216; Lane 3 (CJ-LE):
   Conjunctiva sample from the left eye of patient 0216; Lane 4 (C-RE): Cornea sample from the right eye of patient 0216; Lane 5 (CJ-RE): Conjunctiva sample from the right eye of patient 0216; and Lanes 6, 7, 8: Used as negative controls of the amplification reaction.

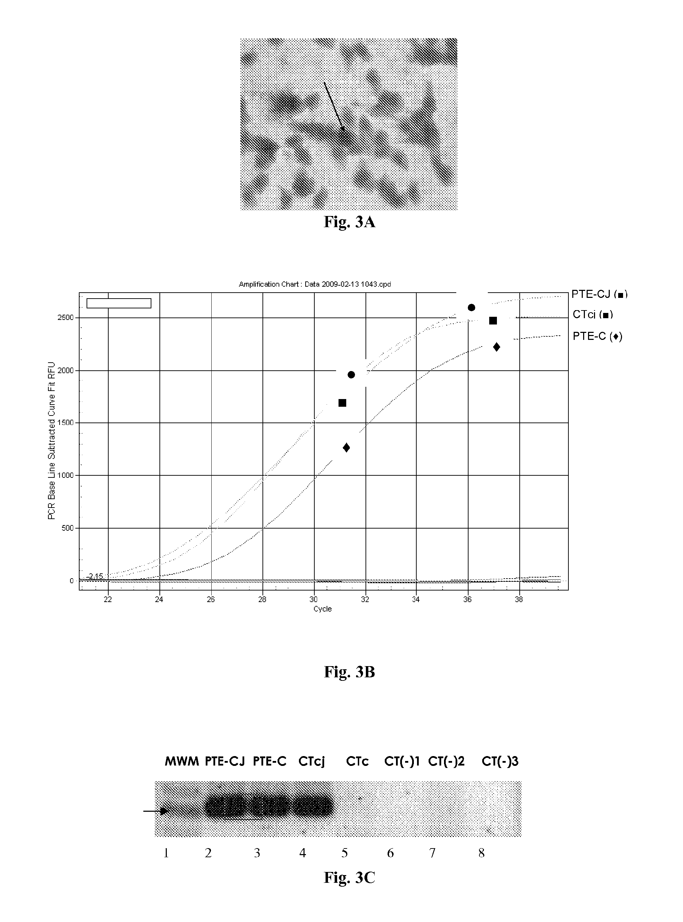

Figure 3 comprises a set of photographs showing a clinical diagnosis of LSCD by impression cytology and PAS-hematoxylin staining (Figure 3A) and by means of RT-qPCR (Figures 3B and 3C) of patient 076. Figure 3A is a microphotograph of corneal cells of patient 076 at 20X; the arrow indicates goblet cells. Figure 3B is a graph showing the amplification curves obtained by means of RT-qPCR of the specific fragment of the *MUC5AC* gene amplified with the pair of oligonucleotide primers formed by the oligonucleotides MUC5AC-RT-F2 (SEQ ID NO: 1) and MUC5AC-RT-R2 (SEQ ID NO: 2) and using a specific fluorescent probe [sequence 5'-CTGGCTGC-3', labeled with 6-FAM at the 5' end] in cornea (PTE-C) and conjunctiva (PTE-CJ) samples from patient 076 and in a conjunctiva sample used as a positive control reference (CTcj). Figure 3C is a photograph of an electrophoresis gel showing the results of the analysis of cornea and conjunctiva samples from patient 076 by means of RT-PCR and subsequent electrophoresis in 2.5% agarose gel and developing with ethidium bromide; lane 3 shows a band corresponding to the specific 103 bp fragment of the mRNA of the *MUC5AC* gene amplified with the pair of oligonucleotide primers formed by the oligonucleotides MUC5AC-RT-F2 (SEQ ID NO: 1) and MUC5AC-RT-R2 (SEQ ID NO: 2), representative of the expression of the *MUC5AC* gene in the analyzed cornea of patient 076. Lane 1 (MWM): Molecular weight marker; Lane 2 (PTE-CJ): Conjunctiva sample from patient 076; Lane 3 (PTE-C): Cornea sample from patient 076; Lane 4 (CTcj): Conjunctiva sample used as a positive control reference; Lane 5 (CTc): Cornea sample used as a negative control reference; and Lanes 6, 7, 8: Used as negative controls of the amplification reaction.

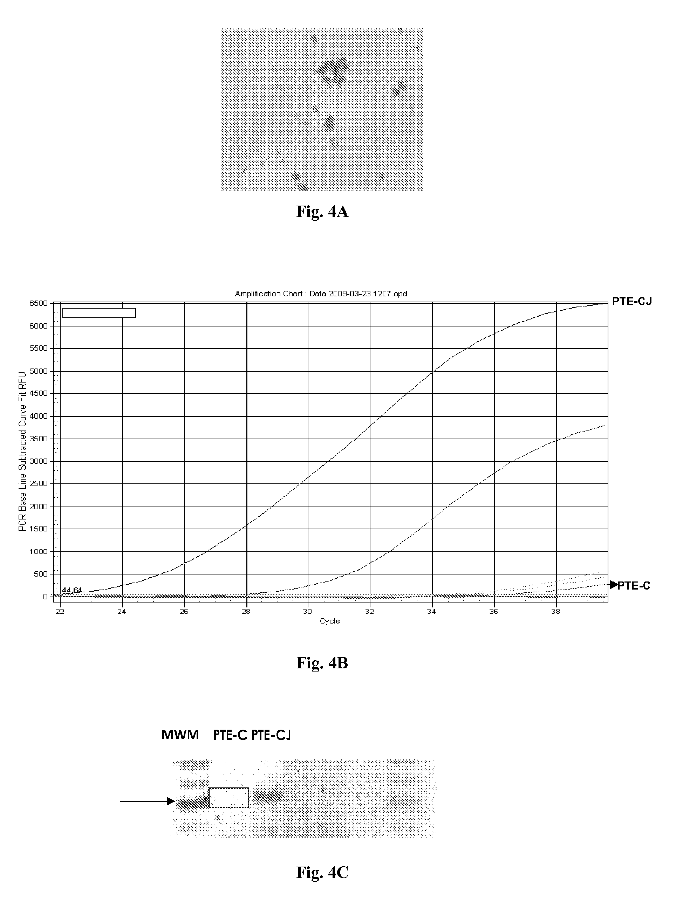

Figure 4 comprises a set of photographs showing a clinical diagnosis of LSCD of patient 0121 which was not confirmed by impression cytology and PAS-hematoxylin staining (Figure 4A) or by means of RT-qPCR (Figures 4B and 4C). Figure 4A is a microphotograph of corneal cells of patient 0121 at 20X. Figure 4B is a graph showing the amplification curves obtained by means of RT-qPCR of the specific fragment of the *MUC5AC* gene amplified with the pair of oligonucleotide primers formed by the oligonucleotides MUC5AC-RT-F2 (SEQ ID NO: 1) and MUC5AC-RT-R2 (SEQ ID NO: 2) and using a specific fluorescent probe [sequence 5'-CTGGCTGC-3', labeled with 6-FAM at the 5' end] in cornea (PTE-C) and conjunctiva (PTE-CJ) samples from patient 0121. Figure 4C is a photograph of an electrophoresis gel showing the results of the analysis of cornea and conjunctiva samples from patient 0121 by means of RT-PCR and subsequent electrophoresis in 2.5% agarose gel and developing with ethidium bromide, wherein the band corresponding to the specific 103 bp fragment of the mRNA of the *MUC5AC* gene in the lane corresponding to the cornea sample from patient 0121 (PTE-C) is not observed, which indicates the non-expression of the *MUC5AC* gene in the analyzed cornea of patient 0121. Lanes 1 (MWM) and 7: Molecular weight markers; Lane 2 (PTE-C): Cornea sample from patient 0121; Lane 3 (PTE-CJ): Conjunctiva sample from patient 0121; and Lanes 5, 5 and 6: Uses as negative controls of the amplification reaction.

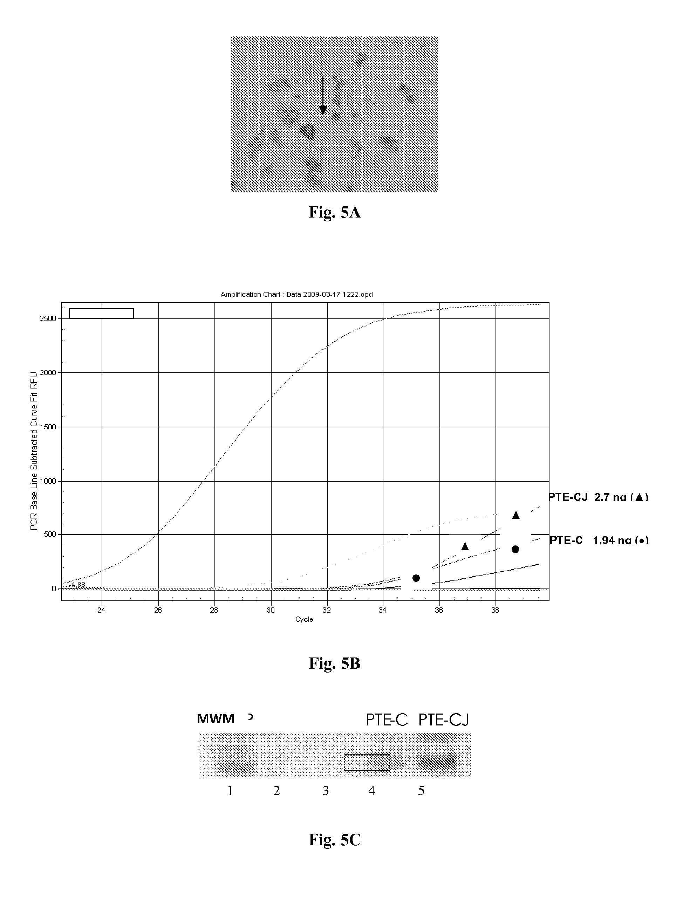

Figure 5 comprises a set of photographs showing a clinical diagnosis of LSCD by impression cytology and PAS-hematoxylin staining (Figure 5A) and by means of RT-qPCR (Figures 5B and 5C) of patient 0123. Figure 5A is a microphotograph of corneal cells of patient 0123 at 20X; the arrow indicates a goblet cell in the analyzed cornea. Figure 5B is a graph showing the amplification curves obtained by means of RT-qPCR of the specific fragment of the *MUC5AC* gene amplified with the pair of oligonucleotide primers formed by the oligonucleotides MUC5AC-RT-F2 (SEQ ID NO: 1) and MUC5AC-RT-R2 (SEQ ID NO: 2) and using a specific fluorescent probe [sequence 5'-CTGGCTGC-3', labeled with 6-FAM at the 5' end] in cornea (PTE-C) and conjunctiva (PTE-CJ) samples from patient 0123. Figure 5C is a photograph of an electrophoresis gel showing the results of the analysis of cornea and conjunctiva samples from patient 0123 by means of RT-PCR and subsequent electrophoresis in 2.5% agarose gel and developing with ethidium bromide; lane 5 shows a band corresponding to the specific 103 bp fragment of the mRNA of the *MUC5AC* gene amplified with the pair of oligonucleotide primers formed by the oligonucleotides MUC5AC-RT-F2 (SEQ ID NO: 1) and MUC5AC-RT-R2 (SEQ ID NO: 2), representative of the expression of the *MUC5AC* gene in the analyzed cornea of patient 0123. Lane 1 (MWM): Molecular weight marker; Lanes 2 and 3: Used as negative controls of the amplification reaction; Lane 4 (PTE-C): Cornea sample from patient 0123; and Lane 5 (PTE-CJ): Conjunctiva sample from patient 0123.

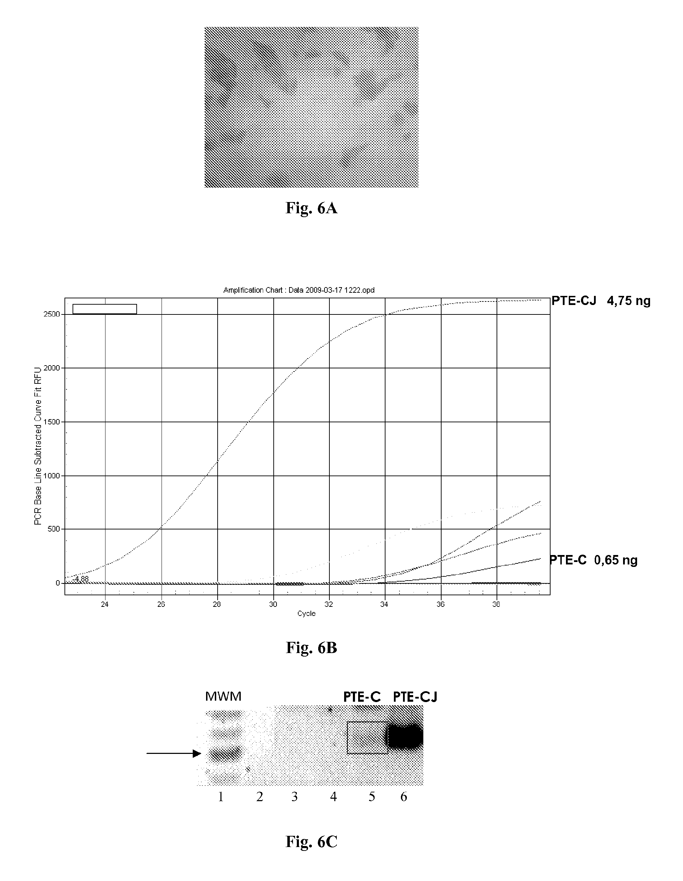

Figure 6 comprises a set of photographs showing a clinical diagnosis of LSCD of patient 0125. Although goblet cells are not seen in the cornea analyzed by impression cytology and PAS-hematoxylin staining (Figure 6A), the presence of the specific 103 bp fragment of the mRNA of the *MUC5AC* gene in the cornea analyzed by RT-qPCR confirms the LSCD (Figure 6C). Figure 6A is a microphotograph of corneal cells of patient 0125 at 20X. Figure 6B is a graph showing the amplification curves obtained by means of RT-qPCR of the specific fragment of the *MUC5AC* gene amplified with the pair of oligonucleotide primers formed by the oligonucleotides MUC5AC-RT-F2 (SEQ ID NO: 1) and MUC5AC-RT-R2 (SEQ ID NO: 2) and using a specific fluorescent probe [sequence 5'-CTGGCTGC-3', labeled with 6-FAM at the 5' end] in cornea (PTE-C) and conjunctiva (PTE-CJ) samples from patient 0125. Figure 6C is a photograph of an electrophoresis gel showing the results of the analysis of cornea and conjunctiva samples from patient 0125 by means of RT-PCR and subsequent electrophoresis in 2.5% agarose gel and developing with ethidium bromide; lane 5 shows a band corresponding to the specific 103 bp fragment of the mRNA of the *MUC5AC* gene amplified with the pair of oligonucleotide primers formed by the oligonucleotides MUC5AC-RT-F2 (SEQ ID NO: 1) and MUC5AC-RT-R2 (SEQ ID NO: 2), representative of the expression of the *MUC5AC* gene in the analyzed cornea of patient 0125. Lane 1 (MWM): Molecular weight marker; Lanes 2, 3 and 4: Used as negative controls of the amplification reaction; Lane 4 (PTE-C): Cornea sample from patient 0125; and Lane 5 (PTE-CJ): Conjunctiva sample from patient 0125.

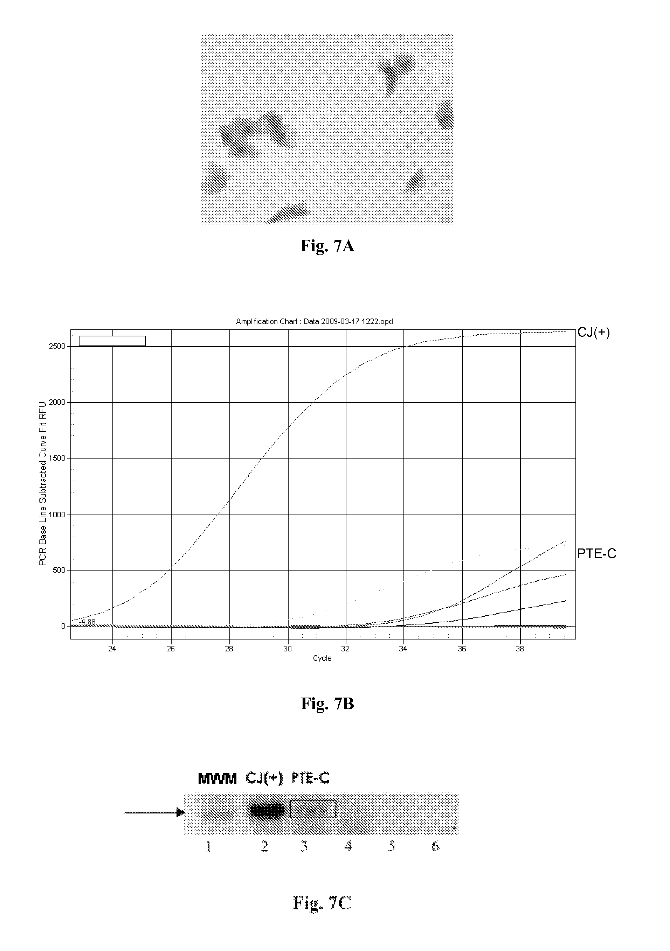

Figure 7 comprises a set of photographs showing a clinical diagnosis of LSCD of patient 0132. Although goblet cells are not seen in the cornea analyzed by impression cytology and PAS-hematoxylin staining (Figure 7A), the presence of the specific 103 bp fragment of the mRNA of the *MUC5AC* gene in the cornea analyzed by RT-qPCR confirms the LSCD (Figure 7C). Figure 7A is a microphotograph of corneal cells of patient 0132 at 20X. Figure 7B is a graph showing the amplification curves obtained by means of RT-qPCR of the specific fragment of the *MUC5AC* gene amplified with the pair of oligonucleotide primers formed by the oligonucleotides MUC5AC-RT-F2 (SEQ ID NO: 1) and MUC5AC-RT-R2 (SEQ ID NO: 2) and using a specific fluorescent probe [sequence 5'-CTGGCTGC-3', labeled with 6-FAM at the 5' end] in cornea (PTE-C) and conjunctiva (CJ(+)) samples from patient 0132. Figure 7C is a photograph of an electrophoresis gel showing the results of the analysis of cornea and conjunctiva samples from patient 0132 by means of RT-PCR and subsequent electrophoresis in 2.5% agarose gel and developing with ethidium bromide; lane 3 shows a band corresponding to the specific 103 bp fragment of the mRNA of the *MUC5AC* gene amplified with the pair of oligonucleotide primers formed by the oligonucleotides MUC5AC-RT-F2 (SEQ ID NO: 1) and MUC5AC-RT-R2 (SEQ ID NO: 2), representative of the expression of the *MUC5AC* gene in the analyzed cornea of patient 0132. Lane 1 (MWM): Molecular weight marker; Lane 2 (CJ(+)): Conjunctiva sample from patient 0132; Lane 3 (PTE-C): Cornea sample from patient 0132; and Lanes 4, 5 and 6: Used as negative controls of the amplification reaction.

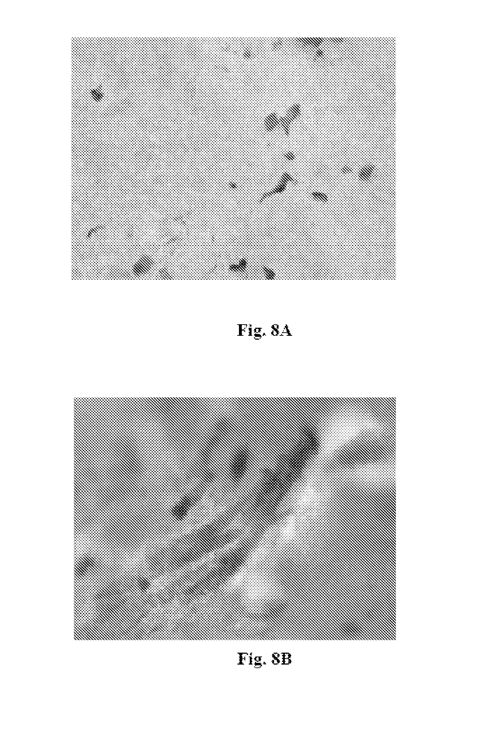

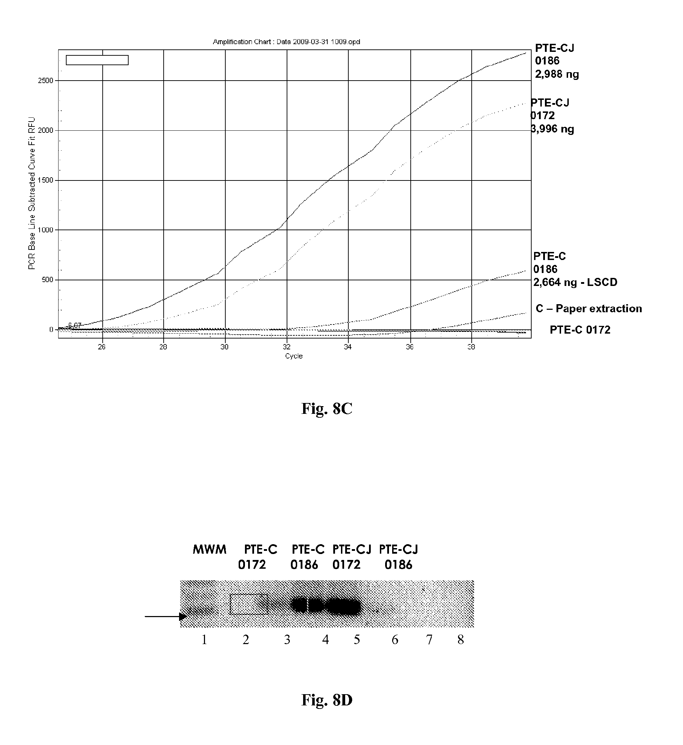

Figure 8 comprises a set of photographs showing a clinical diagnosis of LSCD of patient 0186. Although goblet cells are not seen in the corneas of patients 0172 and 0186 analyzed by impression cytology and PAS-hematoxylin staining (Figure 8A (patient 0172) and Figure 8B (patient 0186)), the presence of the specific 103 bp fragment of the mRNA of the *MUC5AC* gene in the cornea of patient 0186 analyzed by RT-qPCR (Figure 8D) confirms the LSCD in patient 0186. Figures 8A and 8B are microphotographs of corneal cells (20X) of patients 0172 and 0186, respectively. Figure 8B is a graph showing the amplification curves obtained by means of RT-qPCR of the specific fragment of the *MUC5AC* gene amplified with the pair of oligonucleotide primers formed by the oligonucleotides MUC5AC-RT-F2 (SEQ ID NO: 1) and MUC5AC-RT-R2 (SEQ ID NO: 2) and using a specific fluorescent probe [sequence 5'-CTGGCTGC-3', labeled with 6-FAM at the 5' end] in cornea samples (PTE-C) from patient 0186 and conjunctiva samples (PTE-CJ) from patients 0186 and 0172. Figure 8D is a photograph of an electrophoresis gel showing the results of the analysis of cornea and conjunctiva samples from patients 0172 and 0186 by means of RT-PCR and subsequent electrophoresis in 2.5% agarose gel and developing with ethidium bromide; lane 3 shows a band corresponding to the specific 103 bp fragment of the mRNA of the *MUC5AC* gene amplified with the pair of oligonucleotide primers formed by the oligonucleotides MUC5AC-RT-F2 (SEQ ID NO: 1) and MUC5AC-RT-R2 (SEQ ID NO: 2), representative of the expression of the *MUC5AC* gene in the analyzed cornea of patient 0186; said band is not observed in lane 2 corresponding to the analyzed cornea of patient 0172. Lane 1 (MWM): Molecular weight marker; Lane 2 (PTE-C 0172): Cornea sample from patient 0172; Lane 3 (PTE-C 0186): Cornea sample from patient 0186; Lane 4 (PTE-CJ 0172): Conjunctiva sample from patient 0172; Lane 5 (PTE-CJ 0186): Conjunctiva sample from patient 0186; and Lanes 6, 7 and 8: Used as negative controls of the amplification reaction.

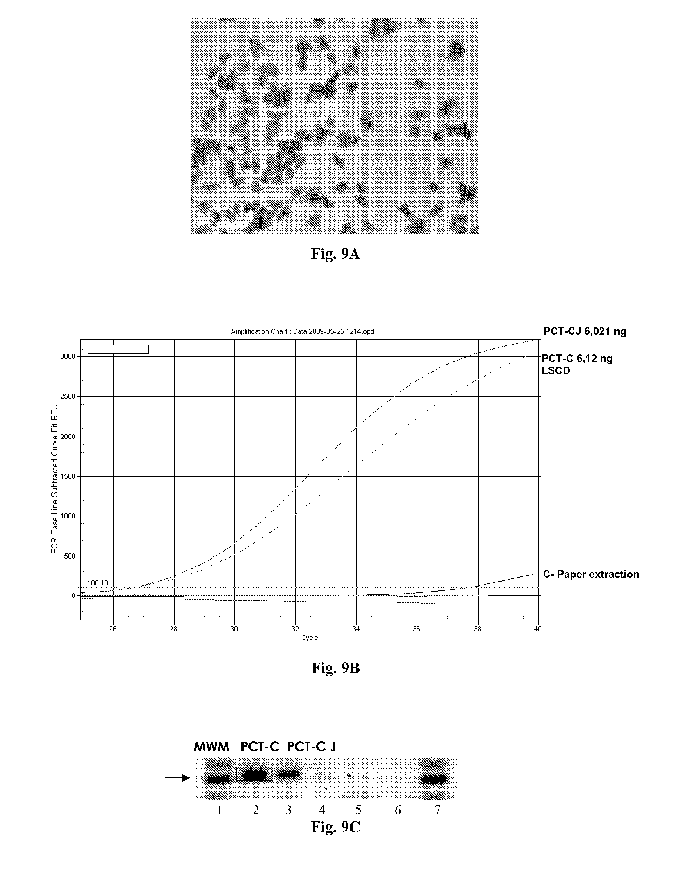

Figure 9 comprises a set of photographs showing a clinical diagnosis of LSCD of patient 0214. Although goblet cells are not seen in the cornea analyzed by impression cytology and PAS-hematoxylin staining (Figure 9A), the presence of the specific 103 bp fragment of the mRNA of the *MUC5AC* gene in the cornea analyzed by RT-qPCR confirms the LSCD (Figure 9C). Figure 9A is a microphotograph of corneal cells of patient 0214 at 20X. Figure 9B is a graph showing the amplification curves obtained by means of RT-qPCR of the specific fragment of the *MUC5AC* gene amplified with the pair of oligonucleotide primers formed by the oligonucleotides MUC5AC-RT-F2 (SEQ ID NO: 1) and MUC5AC-RT-R2 (SEQ ID NO: 2) and using a specific fluorescent probe [sequence 5'-CTGGCTGC-3', labeled with 6-FAM at the 5' end] in cornea (PTE-C) and conjunctiva (PTE-CJ) samples from patient 0214. Figure 9C is a photograph of an electrophoresis gel showing the results of the analysis of cornea and conjunctiva samples from patient 0214 by means of RT-PCR and subsequent electrophoresis in 2.5% agarose gel and developing with ethidium bromide; lane 2 shows a band corresponding to the specific 103 bp fragment of the mRNA of the *MUC5AC* gene amplified with the pair of oligonucleotide primers formed by the oligonucleotides MUC5AC-RT-F2 (SEQ ID NO: 1) and MUC5AC-RT-R2 (SEQ ID NO: 2), representative of the expression of the *MUC5AC* gene in the analyzed cornea of patient 0214. Lanes 1 (MWM) and 7: Molecular weight markers; Lane 2 (PCT-C): Cornea sample from patient 0214; Lane 3 (PCT-CJ): Conjunctiva sample from patient 0214; and Lanes 4, 5 and 6: Used as negative controls of the amplification reaction.

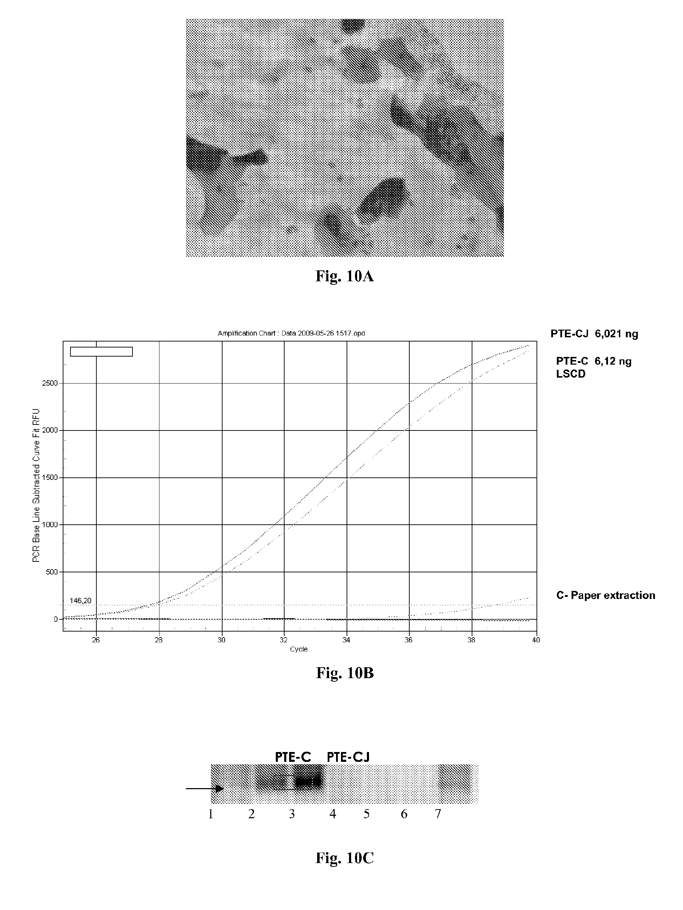

Figure 10 comprises a set of photographs showing a clinical diagnosis of LSCD by impression cytology and PAS-hematoxylin staining (Figure 10A) and by means of RT-qPCR (Figures 10B and 10C) of patient 0233. Figure 10A is a microphotograph of corneal cells of patient 0233 at 20X. Figure 10B is a graph showing the amplification curves obtained by means of RT-qPCR of the specific fragment of the *MUC5AC* gene amplified with the pair of oligonucleotide primers formed by the oligonucleotides MUC5AC-RT-F2 (SEQ ID NO: 1) and MUC5AC-RT-R2 (SEQ ID NO: 2) and using a specific fluorescent probe [sequence 5'-CTGGCTGC-3', labeled with 6-FAM at the 5' end] in cornea (PTE-C) and conjunctiva (PTE-CJ) samples from patient 0233. Figure 10C is a photograph of an electrophoresis gel showing the results of the analysis of cornea and conjunctiva samples from patient 0233 by means of RT-PCR and subsequent electrophoresis in 2.5% agarose gel and developing with ethidium bromide; lane 2 shows a band corresponding to the specific 103 bp fragment of the mRNA of the *MUC5AC* gene amplified with the pair of oligonucleotide primers formed by the oligonucleotides MUC5AC-RT-F2 (SEQ ID NO: 1) and MUC5AC-RT-R2 (SEQ ID NO: 2), representative of the expression of the *MUC5AC* gene in the analyzed cornea of patient 0233. Lanes 1 and 7: Molecular weight markers; Lane 2 (PTE-C): Cornea sample from patient 0233; Lane 3 (PTE-CJ): Conjunctiva sample from patient 0233; and Lanes 4, 5 and 6: Used as negative controls of the amplification reaction.

### Detailed Description of the Invention

Limbal stem cell deficiency (LSCD) is characterized by the existence of conjunctivalization with the presence of goblet cells on the cornea; given that there are no goblet cells in the cornea, the detection of the *MUC5AC* gene in a cornea sample from a subject is indicative of the presence of goblet cells in the cornea and, therefore, of said subject suffering from LSCD.

### Diagnosis of limbal stem cell deficiency (LSCD)

In one aspect, the invention relates to an *in vitro* method for diagnosing limbal stem cell deficiency (LSCD) in a subject, hereinafter "method A of the invention", comprising detecting the expression of the *MUC5AC* gene in a cornea sample from said subject, wherein the detection of expression of the *MUC5AC* gene in said cornea sample is indicative of the subject suffering from LSCD.

The cornea sample can be obtained by conventional methods known by the persons skilled in the art, for example, by means of impression cytology; in a particular embodiment, said cornea sample comprises corneal epithelium tissue.

The expression of the *MUC5AC* gene can be detected by any conventional method suitable for detecting the expression of genes; for example, methods comprising the reverse transcription (RT) of the gene of interest *(MUC5AC)* and the enzymatic amplification of a specific fragment of said gene of interest (*MUC5AC*). Although virtually any method allowing the enzymatic amplification of a specific fragment of the *MUC5AC* gene can be used to put method A of the invention into practice, in a particular embodiment the enzymatic amplification of a specific fragment of the *MUC5AC* gene is carried out by means of the polymerase chain reaction (PCR), in any of its variants.

In a specific embodiment, once the cornea sample from the subject to be analyzed is obtained, the ribonucleic acid (RNA) contained in said sample is extracted. The extraction of the RNA can be carried out by means of any conventional technique known by the persons skilled in the art using, if desired, commercial kits and reagents, e.g., the RNeasy PLUS Micro kit (Qiagen, P/N: 74034). From the extracted RNA, the complementary DNA (cDNA) in relation to the RNA corresponding to the *MUC5AC* gene is synthesized by conventional methods, for example, by means of reverse transcription or retrotranscription (RT) and subsequently a specific fragment of the *MUC5AC* gene is enzymatically amplified by any suitable technique, for example by means of classic or conventional PCR using the suitable oligonucleotide primers; in a particular and preferred embodiment, the enzymatic amplification of a specific fragment of the *MUC5AC* gene is carried out by means of PCR using a pair of oligonucleotide primers formed by an oligonucleotide primer comprising the nucleotide sequence identified as SEQ ID NO: 1 (MUC5AC-RT-F2) and by an oligonucleotide primer comprising the nucleotide sequence identified as SEQ ID NO: 2 (MUC5AC-RT-R2) under conditions which allow amplifying a specific 103 base pair (bp) fragment of the transcript of the *MUC5AC* gene. The use of said oligonucleotide primers is crucial for the high specificity of the method provided by this invention since said oligonucleotide primers exclusively amplify in a specific manner retrotranscribed cDNA of the *MUC5AC* gene but not of the *MUC5B* gene. The expression of the specific fragment of the *MUC5AC* gene can be detected by means of conventional methods, for example, by means of separation (e.g., by agarose gel electrophoresis, etc.) and developing of the amplification product (e.g., by ethidium bromide staining, etc.).

The protocol followed to carry out PCR is widely known in the state of the art and currently there are commercial kits containing the materials necessary for carrying out said amplification. Likewise, the conditions of temperature, time, concentrations of reagents and number of PCR cycles will depend on the DNA polymerase used in the amplification reaction, on the specificity, length and composition of the primers, and the length and composition of the sequence. If a commercial kit is used, the conditions of the reaction will be those specified by the manufacturer of the kit.

In the event that the expression level of the *MUC5AC* gene in a cornea sample from the subject is to be quantified, the enzymatic amplification reaction of the specific fragment of said *MUC5AC* gene can be carried out by means of a quantitative PCR (qPCR) or "real-time PCR", as described in more detail below in relation to "method B of the invention".

The detection of the *MUC5AC* gene in a cornea sample from a subject is indicative of the presence of goblet cells in the cornea, and, consequently, of said subject suffering from LSCD.

Method A of the invention has a number of advantages since it is a non-invasive method, with high sensitivity and specificity (since the fragment amplified by using the pair of oligonucleotide primers formed by the oligonucleotide primers comprising the sequences identified as SEQ ID NO: 1 and SEQ ID NO: 2 comprises a concrete and specific region of the *MUC5AC* gene), high reproducibility and, furthermore, enables the identification of erroneous clinical diagnoses.

Said oligonucleotides comprising the nucleotide sequences shown in SEQ ID NO: 1 (MUC5AC-RT-F2) and in SEQ ID NO: 2 (MUC5AC-RT-R2) and the combinations thereof, as well as the use thereof in the diagnosis of LSCD are an additional aspect of this invention as described in detail below.

The reagents necessary for putting method A of the invention into practice can be included in a kit. Said kit and its use in the diagnosis of LSCD are additional aspects of this invention which will be described in detail below.

In another aspect, the invention relates to an *in vitro* method for diagnosing limbal stem cell deficiency (LSCD) in a subject, hereinafter "method B of the invention", comprising:
a) quantifying the expression of the *MUC5AC* gene in a cornea sample from said subject; and
b) comparing the expression level of the *MUC5AC* gene detected in step (a) with the expression level of the *MUC5AC* gene in a reference sample;
wherein an increase in the expression level of the *MUC5AC* gene detected in step (a) with respect to the expression level of the *MUC5AC* gene in the reference sample is indicative of LSCD.

In order to put it into practice, method B of the invention comprises obtaining a cornea sample from the subject (test sample) to be analyzed. The reference sample is a sample of a tissue which does not express the *MUC5AC* gene, for example, corneal epithelial tissue, since healthy cornea lacks goblet cells and, therefore, there is no expression of the *MUC5AC* gene; consequently, said reference sample acts as a negative control of expression of the *MUC5AC* gene. Additionally, if desired, a sample of tissue expressing the *MUC5AC* gene, for example, conjunctival epithelial tissue since the conjunctiva contains goblet cells expressing the *MUC5AC* gene, can be incorporated as a positive control. The cornea samples (test sample and reference sample), like the conjunctiva sample (positive control of expression of the *MUC5AC* gene) can be obtained by conventional methods known by the persons skilled in the art, for example, by means of impression cytology (see Example 1.2). In a particular embodiment, both cornea samples (test sample and reference sample) comprise corneal epithelium tissue. Likewise, in a particular embodiment, the conjunctiva sample (in the event of being used) comprises conjunctival epithelium cells. All the samples (test, reference or negative control and, optionally, positive control of expression of the *MUC5AC* gene) are separately processed in the same manner.

The quantification of the expression of the *MUC5AC* gene can be carried out by any suitable method for the quantification of the expression of a gene; for example, by means of a method comprising the reverse transcription (RT) of the gene of interest (*MUC5AC*), the enzymatic amplification of a specific fragment of said gene of interest *(MUC5AC)* and its quantification. In a particular embodiment, the amplification and quantification of the expression of the *MUC5AC* gene is carried out by means of PCR; although a classic or conventional PCR can be used, in practice the use of a quantitative PCR (qPCR) or "real-time PCR" is preferred since it allows amplifying and simultaneously quantifying the DNA amplification product using the suitable oligonucleotide primers and a substance labeled with a fluorophore since classic PCR requires carrying out additional steps (separation, staining and preparation of standards) which complicate the process. Any qPCR technique can be used, both the techniques based on unspecific fluorochromes (e.g., techniques based on using fluorochromes which bind specifically to double-stranded DNA such as SYBR Green, etc.), and the techniques based on using specific probes (e.g., Taqman type probes, Molecular Beacon type probes, Scorpion type probes, etc). Therefore, in a particular and preferred embodiment, the quantification of the expression of the *MUC5AC* gene is carried out by means of RT-qPCR; in Example 1 attached to this description, the quantification of the expression of the *MUC5AC* gene is carried out by means of RT-qPCR using a Taqman type probe.

To quantify the expression of the *MUC5AC* gene, in a particular embodiment, the RNA contained in the samples (test, reference and, optionally, positive control) is extracted. The extraction of the RNA can be carried out by means of any technique allowing the extraction of RNA, for example, by using, if desired, commercial kits and reagents, e.g., the RNeasy PLUS Micro kit (Qiagen, P/N: 74034). From the extracted RNA, the complementary DNA (cDNA) in relation to the RNA corresponding to the *MUC5AC* gene is synthesized by conventional methods, for example, by means of RT using the suitable reagents and conditions, and, subsequently, the enzymatic amplification of a specific fragment of the *MUC5AC* gene and the simultaneous quantification of the expression of said *MUC5AC* gene are carried out using the suitable oligonucleotide primers, dNTPs, a suitable reaction buffer and a heat-stable DNA polymerase, and a substance labeled with a fluorophore, e.g., a Taqman type probe is incorporated to the reaction mixture formed. In a particular and preferred embodiment, the enzymatic amplification of a specific fragment of the *MUC5AC* gene and its simultaneous quantification is carried out by means of a qPCR using a pair of oligonucleotide primers formed by an oligonucleotide primer comprising the nucleotide sequence identified as SEQ ID NO: 1 (MUC5AC-RT-F2) and by an oligonucleotide primer comprising the nucleotide sequence identified as SEQ ID NO: 2 (MUC5AC-RT-R2) [the use of this pair of oligonucleotides allows specifically amplifying a fragment of retrotranscribed cDNA of *MUC5AC*] under conditions which allow amplifying a specific 103 base pair (bp) fragment of the transcript of the *MUC5AC* gene, in the presence of a substance labeled with a fluorophore, such as a fluorochrome which binds specifically to double-stranded DNA (e.g., SYBR Green, etc.), or a specific probe (e.g., a Taqman type, or Molecular Beacon type, or Scorpion type probe, etc); in a particular embodiment, the quantification of the expression of the *MUC5AC* gene is carried out by means of RT-qPCR using a specific Taqman type probe which has the nucleotide sequence 5'-CTGGCTGC-3' and is labeled at the 5' end with the fluorophore 6-FAM (6-carboxyfluorescein), compatible for the detection thereof in real-time qPCR equipment (Example 1.3).

The use of said pair of oligonucleotides [oligonucleotides comprising the nucleotide sequences shown in SEQ ID NO: 1 (MUC5AC-RT-F2) and in SEQ ID NO: 2 (MUC5AC-RT-R2)] is crucial for the high specificity of the method of the invention since said primers exclusively amplify in a specific manner retrotranscribed cDNA of *MUC5AC* (in no case *MUC5B*).

The protocol followed to carry out the RT-qPCR is widely known in the state of the art and currently there are commercial kits containing the materials necessary for carrying out said amplification. Likewise, the conditions of temperature, time, reagents, concentrations of reagents and number of PCR cycles will depend on the DNA polymerase used in the amplification reaction, on the specificity, length and composition of the primers, and the length and composition of the sequence. If a commercial kit is used, the conditions of the reaction will be those specified by the manufacturer of the kit. The reagents and the conditions used to put method B of the invention into practice are shown in Example 1 attached to this description. In a particular embodiment, method B of the invention is carried out using the reaction mixture described in Example 1; nevertheless, any other suitable combination of reaction mixture can be used.

Method B of the invention comprises, in addition to quantifying the expression of the *MUC5AC* gene in a cornea sample from the subject that is subjected to analysis [step a)], comparing the expression level of the *MUC5AC* gene detected in step (a) with the expression level of the *MUC5AC* gene in a reference sample [step b)] to establish if the subject under study suffers from LSCD.

In a specific embodiment, the reference sample is a healthy cornea sample, i.e., from a subject who does not suffer from LSCD. Alternatively, the expression level of the *MUC5AC* gene in a reference sample can be established as a reference value and can be obtained from the values of expression of the *MUC5AC* gene obtained from samples of corneal epithelial tissue obtained from one or more, for example, 2 or more, 10 or more, 20 or more, etc., subjects who do not suffer from LSCD. It would thus not be necessary to obtain a reference cornea sample or to quantify the expression of the *MUC5AC* gene in said reference sample every time an assay for diagnosing LSCD is carried out, which would simplify the work and save costs.

Once the expression level of the MUC5AC gene in the sample from the subject to be analyzed has been obtained and the expression level of the *MUC5AC* gene in a reference sample or the reference value of the expression of said MUC5AC gene in a reference sample has been determined, the expression level of the *MUC5AC* gene detected in the test sample is compared with the expression level of the *MUC5AC* gene in the reference sample or with the reference value to determine if has or has not been an increase in the expression level of the *MUC5AC* gene detected in said test sample with respect to the expression level of the *MUC5AC* gene in the reference sample or with respect to the reference value. An increase in the expression of said MUC5AC gene in the test sample under study is considered as a "significant increase" with respect to the expression level of the *MUC5AC* gene in the reference sample or with respect to the reference value when the expression level in the test sample increases with respect to the expression level of the *MUC5AC* gene in the reference sample or with respect to the reference value by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or even more. As has been previously mentioned, an increase in the expression level of the *MUC5AC* gene in a cornea sample from the subject under study with respect to the expression level of the *MUC5AC* gene in the reference sample or with respect to the reference value is indicative of said subject suffering from LSCD.

Additionally and optionally, if desired, the expression level of the *MUC5AC* gene in a sample of tissue expressing the *MUC5AC* gene, for example, conjunctival epithelial tissue since the conjunctiva contains goblet cells expressing the *MUC5AC* gene, can be quantified and said sample can be used as a positive control of amplification of said *MUC5AC* gene.

In a particular and preferred embodiment of method B of the invention, the invention provides an *in vitro* method for diagnosing LSCD in a subject, comprising:
a) quantifying the expression of the *MUC5AC* gene by means of RT-qPCR in a cornea sample from said subject, using a pair of oligonucleotide primers formed by an oligonucleotide primer comprising the nucleotide sequence identified as SEQ ID NO: 1 (MUC5AC-RT-F2) and by an oligonucleotide primer comprising the nucleotide sequence identified as SEQ ID NO: 2 (MUC5AC-RT-R2), under conditions which allow amplifying a specific fragment of the *MUC5AC* gene; and
b) comparing the expression level of the *MUC5AC* gene detected in step (a) with the expression level of the *MUC5AC* gene in a reference sample;
wherein an increase in the expression level of the *MUC5AC* gene detected in step (a) with respect to the expression level of the *MUC5AC* gene in the reference sample is indicative of LSCD.

A healthy cornea sample, i.e., from a subject who does not suffer from LSCD, can be used as a reference sample. Alternatively, the expression level of the *MUC5AC* gene determined in step a) can be compared with the reference value of expression of the *MUC5AC* gene in healthy cornea obtained from the values of expression of the *MUC5AC* gene obtained from samples of corneal epithelial tissue obtained from one or more subjects who do not suffer from LSCD. Additionally, if desired, the expression level of the *MUC5AC* gene in a sample of tissue expressing the *MUC5AC* gene, for example, conjunctival epithelial tissue, can be quantified and said sample can be used as a positive control of amplification of said *MUC5AC* gene.

Method B of the invention has a number of advantages since it is a non-invasive method which allows obtaining very reliable quantitative data, with high sensitivity and specificity (since the fragment amplified by using the pair of oligonucleotide primers formed by the oligonucleotide primers comprising the sequences identified as SEQ ID NO: 1 and SEQ ID NO: 2 comprise a concrete and specific region of the *MUC5AC* gene), high reproducibility and, furthermore, enables the identification of erroneous clinical diagnoses.

### Oligonucleotide primers

As has been previously mentioned, in another aspect, the invention relates to an oligonucleotide selected from the group consisting of an oligonucleotide comprising the nucleotide sequence shown in SEQ ID NO: 1 (MUC5AC-RT-F2), an oligonucleotide comprising the nucleotide sequence shown in SEQ ID NO: 2 (MUC5AC-RT-F2) and mixtures thereof.

When said oligonucleotides are used as oligonucleotide primers in a PCR, such as a classic PCR or a variant thereof (e.g., qPCR), they allow amplifying a concrete and specific region or fragment of the *MUC5AC* gene but not of other mucin genes (e.g., *MUC5B*), therefore they can be used to enzymatically amplify, for example, by means of PCR, a specific fragment of the *MUC5AC* gene, or to detect and identify said *MUC5AC* gene, or to quantify said *MUC5AC* gene, or in the diagnosis of LSCD by means of detecting and/or quantifying the *MUC5AC* gene by PCR (e.g., classic PCR or qPCR).

### Kits

The reagents necessary for putting both method A of the invention and method B of the invention into practice can be included in a kit. Said kit and its use in the diagnosis of LSCD are additional aspects of this invention which will be described in detail below.

Therefore, in another aspect, the invention relates to a kit, hereinafter kit of the invention, comprising an oligonucleotide selected from the group consisting of an oligonucleotide comprising the nucleotide sequence shown in SEQ ID NO: 1 (MUC5AC-RT-F2), an oligonucleotide comprising the nucleotide sequence shown in SEQ ID NO: 2 (MUC5AC-RT-F2) and mixtures thereof.

In a particular embodiment, said kit of the invention comprises at least the pair of oligonucleotide primers formed by an oligonucleotide primer comprising SEQ ID NO: 1 (MUC5AC-RT-F2) and an oligonucleotide primer comprising SEQ ID NO: 2 (MUC5AC-RT-R2). A kit of this type is particularly useful for detecting the expression of the *MUC5AC* gene by means of PCR and, consequently, said kit can be used in the diagnosis of LSCD in a subject.

In another particular embodiment, said kit of the invention furthermore comprises a substance labeled with a fluorophore, such as a fluorochrome which binds specifically to double-stranded DNA (e.g., SYBR Green, etc.), or a probe specific for the *MUC5AC* gene (e.g., a Taqman type probe, a Molecular Beacon type probe, a Scorpion type probe, etc.); in a specific embodiment, said substance labeled with a fluorophore is a Taqman type probe specific for the *MUC5AC* gene amplified by the oligonucleotide primers provided by this invention, such as the probe (Probe #71) (Roche) which has the nucleotide sequence 5'-CTGGCTGC-3' and is labeled at the 5' end with the fluorophore 6-FAM. A kit of this type is particularly useful for quantifying the expression of the *MUC5AC* gene by means of qPCR and, consequently, said kit can be used in the diagnosis of LSCD in a subject.

### Selection of suitable patients for corneal transplantation or keratoplasty

As is known, some types of blindness can be caused by an opacity or loss of the optical properties of the cornea; in some cases the remedy thereof is possible by means of corneal transplantation or keratoplasty, i.e., replacing the damaged part of the cornea with a fragment of healthy and transparent cornea from a donor.

For the keratoplasty to be successful, it is necessary for the tissue bed of the recipient to be healthy. In a conventional central keratoplasty the epithelial cells of the donor button are usually limited to 12-18 months [Kinoshita et al., Invest Ophthalmol Vis Sci (1981), 21(3):434-41]. However, if the corneal and limbal peripheral epithelium of the recipient is compromised, the performance of the keratoplasty would assure a competent epithelium **only** while the cells thereof survive. When the latter are replaced with the peripheral corneal epithelium of the recipient or with the conjunctival one if the limbal barrier is not competent, the typical manifestations of the disease of fundamental corneal cells would become evident with the onset of persistent epithelial defects, chronic inflammation and tendency to neovascularization which would compromise the transparency of the graft and its future transplantation.

It would therefore be convenient to have a method which allows classifying or selecting patients with a corneal pathology who can be subjected to keratoplasty with reasonable expectations of success for the purpose of reducing or minimizing the risk of failure of said technique.

The invention provides a solution to said need based on detecting and/or quantifying the *MUC5AC* gene in a cornea sample from said patient with a corneal pathology susceptible to keratoplasty such that, if said *MUC5AC* gene is detected in a cornea sample from said patient, or if the expression of said *MUC5AC* gene in a cornea sample from said patient is greater than a reference value of the expression of the MUC5AC gene in a reference sample (e.g., healthy cornea), then said patient suffers from LSCD and, therefore, the keratoplasty would be ineffective since it would not allow remedying said corneal pathology. In this case, said patient who suffers from LSCD should be treated by means of a suitable treatment, for example, by means of limbal stem cell transplantation. Limbal stem cell transplantation consists of partially or completely substituting the limbus of the recipient with a healthy limbus, normally an autotransplant (from the contralateral healthy eye) or an allotransplant (from a donor), the latter must be associated with systemic immunosuppression. Limbal stem cell transplantation has been proposed as an effective measure for restoring the epithelial integrity of the patient [Kenyon KR & Tseng SC. Ophthalmology (1989), 96:709-723; Thoft RA. Ophthalmology (1982), 89:335; Thoft RA. Am J Ophthalmology (1984), 97:1-6).

Therefore, in another aspect, the invention relates to a method for evaluating if a patient with a corneal pathology susceptible to keratoplasty is a suitable candidate for keratoplasty, comprising:
- detecting the expression of the *MUC5AC* gene in a cornea sample from said patient; or, alternatively
- determining the expression level of the *MUC5AC* gene in a cornea sample from said patient; and
if the *MUC5AC* gene is not detected in said cornea sample from said patient; or, alternatively, if the expression level of the *MUC5AC* gene detected in the cornea sample from said patient is equal to the expression level of the *MUC5AC* gene in a reference sample, then said patient is a suitable candidate for keratoplasty.

As used herein, the term "corneal pathologies susceptible to keratoplasty" includes aniridia, keratitis associated with multiple endocrine deficiencies, neurotrophic keratopathy, pterygium and pseudopterygium, chemical and thermal injuries, Stevens-Johnson syndrome, multiple surgeries or cryotherapies in the limbal region, keratopathy induced by contact lenses, caustications, hypovitaminosis and herpes, among others [Tseng SCG. Eye (1989), 3:141-157; Mendicute et al. Arch Soc Esp Oftalmol (1994), 66:435-442; Barraquer et al. Atlas de microcirugia de la cornea, 1982].

In this case, the reference sample is a healthy cornea sample. The characteristics of the cornea sample from the patient and of the reference sample (healthy cornea) as well as the manner of obtaining them, and the methodology for detecting and/or quantifying the expression of the *MUC5AC* gene in said cornea samples have already been mentioned previously in relation to the diagnosis of LSCD. Likewise, as has been previously mentioned in relation to method B of the invention, the quantification of the expression level in the reference sample can be replaced with a reference value of the expression of the *MUC5AC* gene in healthy cornea (reference value in this case) determined from the values of expression of the *MUC5AC* gene in samples of corneal epithelial tissue obtained from one or more, for example, 2 or more, 10 or more, 20 or more, etc., subjects who do not suffer from LSCD. It would thus not be necessary to obtain a reference cornea (healthy cornea) sample or to quantify the expression of the *MUC5AC* gene in said reference sample every time it is to be evaluated if a patient with a corneal pathology susceptible to keratoplasty is a suitable candidate for keratoplasty according to the present invention, which would simplify the work and save costs.

According to the previously described method, if the *MUC5AC* gene is not detected in said cornea sample from said patient; or, alternatively, if the expression level of the *MUC5AC* gene detected in the cornea sample from said patient is equal to the expression level of the *MUC5AC* gene in a reference sample or to the reference value, then said patient is a suitable candidate for keratoplasty since said patient does not suffer from LSCD, which could cause the failure of the keratoplasty.

In another aspect, the invention relates to a method for evaluating if a patient with a corneal pathology susceptible to keratoplasty is a suitable candidate for keratoplasty which comprises subjecting said patient to an assay for diagnosing if he or she suffers from limbal stem cell deficiency (LSCD), and, if said patient does not suffer from LSCD, then said patient is a suitable candidate for keratoplasty. As has been previously mentioned, if the patient suffers from LSCD, then the keratoplasty would be ineffective since said pathology would cause the failure of the keratoplasty. In a particular embodiment, said assay for diagnosing if said patient with a corneal pathology susceptible to keratoplasty suffers from LSCD is carried out by means of any of methods A or B for diagnosing LSCD provided by this invention.

The invention is illustrated below based on the following examples which are provided by way of a non-limiting illustration of the scope of the invention.

### EXAMPLE 1

### Evaluation of the expression of the MUC5AC gene in the cornea as a marker for the diagnosis of limbal stem cell deficiency by means of RT-qPCR

### Materials and Methods

### 1.1 Characterization of the MUC5AC gene, fragment of mRNA to be amplified and design of oligonucleotide primers

The human *MUC5AC* gene (NM_017511) is a 112.8 kb gene, located in chromosome 11, the coordinates of which are chr11:1,132,474-1,245,302 (11p15.5). For the purpose of designing the oligonucleotide primers, the complementary reverse sequence of the RNA of the *MUC5AC* gene was used, since the real-time PCR is carried out on cDNA. To obtain specificity data, the BLAT program (Kent Informatics, Inc) has been used for the RNA to be amplified. By operating in this manner, the pair of optimal oligonucleotide primers for detecting the *MUC5AC* gene has been designed, the pair being formed by the forward oligonucleotide primer identified as MUC5AC-RT-F2 (SEQ ID NO: 1) and by the reverse oligonucleotide primer identified as MUC5AC-RT-R2 (SEQ ID NO: 2): The probe specific for the amplified fragment used in the qPCR has the nucleotide sequence 5'-CTGGCTGC-3' and is labeled with 6-FAM at the 5' end.

### 1.2 Samples used

Samples were collected from patients selected from outpatient services of the Hospital de Cruces of Bilbao and of the Hospital Donostia of San Sebastián. The inclusion criteria were established by means of anamnesis and clinical assessment and included patients with suspected LSCD. A patient was considered to present signs of LSCD when in the slit lamp examination the patient presented a corneal vascularization as well as signs of corneal erosions and permanent instability of the tear film.

A cornea sample and a conjunctiva sample were taken from the selected patients by means of impression cytology for their analysis by means of RT-qPCR. Furthermore, a cornea sample and another conjunctiva sample from the same eye were also taken from them by impression cytology, to analyze them by means of PAS-hematoxylin staining. Simultaneously, a cornea sample from a subject without LSCD was taken by means of impression cytology. The effectiveness of both methods (PAS-hematoxylin staining and RT-qPCR) can thus be evaluated and the reference samples can be obtained to compare the values at the time of the quantification.

For the collection of samples, circular membranes with a size considerably smaller than the diameter of the cornea for the extraction of RNA, and rectangular membranes with a peak at their end (5 x 5 mm) for a correct orientation for the PAS-hematoxylin staining were used. In both cases the same paper was used, Millipore nitrocellulose paper (HAWP304).

The impression cytology samples intended for the extraction of RNA were stored in microtubes containing "RNAprotect cell reagent" (QIAgen P/N: 76526), for the purpose of protecting the RNA and so that the samples could be preserved at room temperature until their analysis, preventing their degradation. For the PAS-hematoxylin staining, the impression cytology samples were fixed with 96% ethanol and subsequently stained with PAS-hematoxylin. The evaluation was carried out by means of optical microscopy.

### 1.3 Extraction and quantification of RNA

The RNeasy PLUS Micro kit (Qiagen, P/N: 74034) was used for the extraction of RNA, and all the steps were carried out at room temperature (15-25°C). Briefly, the membrane containing the sample was transferred to a new RNAse-free tube and shaken for 10 seconds at maximum speed to eliminate the surplus. The membrane was again transferred to another tube and 75 µl of the Buffer RLT Plus [Qiagen] were added. The cells were lysed by means of shaking for 2 minutes. The lysate and the membrane were transferred to a "gDNA Eliminator" column [Qiagen] and centrifuged for 30 seconds at ≥ 8,000 g (≥10,000 rpm). A volume of 70% ethanol (75 µl) was added to the resulting supernatant and the mixture was stirred. The entire volume (including any precipitate formed) was transferred to an "RNeasy MinElute" column [Qiagen] with its collection tube and centrifuged for 15 seconds at ≥ 8,000 g (≥10,000 rpm). The supernatant and the collection tube were discarded and 700 µl of Buffer RW1 [Qiagen] were added to the column and centrifuged for 15 seconds at ≥ 8,000 g (≥10,000 rpm). Subsequently, 500 µl of buffer RPE [Qiagen] were added to the column and it was centrifuged again. The column was washed with 500 µl of 80% ethanol. Finally, it was eluted in a new tube with 22 µl of RNAse-free water in the center of the column and incubated for 10 minutes at room temperature in order to then centrifuge for 1 minute at maximum speed. The RNA obtained can be preserved at -80°C for 1 year.

### 1.4 Retrotranscription (RT) reaction

For the retrotranscription of RNA, the "Transcriptor First Strand cDNA Synthesis" kit, from Roche (P/N: 04 379 012 001), with a combination of Oligo(dT)18 and Random Hexamers as primers of the reaction, was used.

Table 1 indicates the composition of the reaction mixture for the standard retrotranscription of *MUC5AC.* The volume of RNA can vary, depending on the concentration obtained in the extraction.

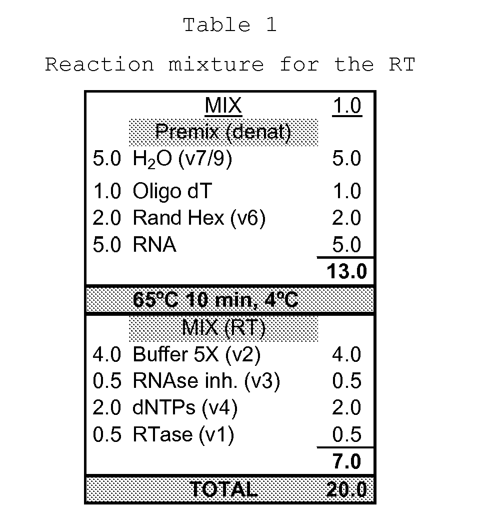

The first step of the reaction consisted of an initial denaturation before adding the rest of the reagents (of the second part of the mixture). Once the 10 minutes have elapsed, the rest of the reagents were added and the retrotranscription was carried out. The retrotranscription cycles were: 10 minutes at 25°C, 30 minutes at 55°C, 5 minutes at 85°C and finally it was maintained at 4°C.

The cDNA obtained at the end of the protocol could be used directly, without prior purification as a template for the PCR.

### 1.5 Real-time PCR (qPCR)

2 µl of non-purified retrotranscription product (cDNA) were used as a template for the qPCR. To that end, the following oligonucleotide primers were used:
SEQ ID NO: 1 (MUC5AC-RT-F2) and
SEQ ID NO: 2 (MUC5AC-RT-R2)
and the Probe #71 from Roche, specific for the amplification product, which has the nucleotide sequence 5'-CTGGCTGC-3' and is labeled at the 5' end with the fluorophore 6-FAM, compatible for the detection thereof in real-time PCR equipment.

The reaction mixture was prepared with a final volume of 20 µl with the amounts indicated in Table 2.

**Table 2**

| Reaction mixture of the qPCR | | | |
|---|---|---|---|
| | MIX | 1 | |
| 10.760 | H₂O | 10.76 | |
| 2 | Buffer 10X | 2 | |
| 1.4 | 50 mM MgCl₂ | 1.4 | |
| 0.64 | 25 mM dNTPs | 0.64 | |
| 1.2 | F primer | 1.2 | |
| 1.2 | R primer | 1.2 | |
| 0.6 | Probe #71 | 0.6 | |
| 0.2 | Taq | 1.2 | |
| | | 18 | µl MIX + 2µl cDNA |

| | | | |
|---|---|---|---|
| F primer: SEQ ID NO: 1 (MUC5AC-RT-F2) R primer: SEQ ID NO: 2 (MUC5AC-RT-R2) Probe #71 (Roche): 5'-CTGGCTGC-3' labeled at the 5' end with 6-FAM. | | | |

The relative quantification of the amplifications was analyzed using the "iQ5 optical system" program included in the iQ5 Q-PCR equipment (Biorad). The conditions of the PCR are indicated in Table 3.

**Table 3**

| qPCR conditions | | | | | |
|---|---|---|---|---|---|
| Step 1 | Step 2 | | | Step 3 | |
| | | X40 | | | |
| | | | 72°C | | |
| 96°C | 96°C | 60°C | (RT) | 72°C | 15°C |
| 3 min | 20 s | 20 s | 10 s | 5 min | ∞ |

### Results

A prospective study has been conducted on 14 eyes of 13 patients (7 men and 6 women) with limbal stem cell deficiency caused by ocular burns (2 eyes), Stevens-Johnson syndrome (2 eyes), ocular cicatricial pemphigoid (2 eyes), contact lens wearers (1 eye), others (7). The study inclusion criteria were established by means of anamnesis and clinical assessment. A patient was considered to present signs of limbal stem cell deficiency when in the slit lamp examination the patient presented a corneal vascularization as well as signs of corneal erosions and permanent instability of the tear film.

The analyzed cornea and conjunctiva samples were collected by means of impression cytology and processes as has been indicated in the previous sections.

The transcript of the mucin gene (*MUC5AC*) was detected in 10 of the 14 corneal epithelium samples analyzed by RT-PCR. However, it was only possible to diagnose LSCD by means of conventional impression cytology with PAS-hematoxylin in 5 patients analyzed.

The results obtained are shown in Figures 1-10 (corresponding to Cases 1-13 mentioned below).

### CASES 1, 2 and 3: Samples from patients 5, 6 and 7

Figure 1 shows the results of the analysis of patients 5, 6 and 7 in one and the same gel by means of detecting the *MUC5AC* gene. In these cases, the results corresponding to the impression cytology and PAS-hematoxylin staining are not available. As can be seen in said Figure 1, LSCD is confirmed for patient 5 but not for patients 6 and 7 by means of amplifying the specific 103 bp fragment of the mRNA of the *MUC5AC* gene.

The results obtained in the patients analyzed by comparing the diagnoses of the impression cytology and PAS-hematoxylin staining with those obtained by means of amplifying a specific 103 bp fragment of the mRNA of the *MUC5AC* gene by RT-qPCR are shown below.

### CASE 4: Sample from patient 0216

Patient with LSCD, diagnosed by slit lamp (Figures 2A and 2B), by impression cytology and PAS-hematoxylin staining (Figures 2C and 2D) and by means of RT-qPCR (Figures 2E and 2F). The presence of goblet cells in the cornea is observed (Figures 2C and 2D). Figure 2E shows values around zero (non-amplification) or negative values and the positive values (*) between 500 and 1,000 RFU (relative fluorescence units). The curve (C1) corresponds to the cornea sample from the left eye of lane 2 in the agarose gel (Figure 2F) and the curve (E1) corresponds to the cornea sample from the right eye of lane 4 in the agarose gel (Figure 2F). As can be seen in said Figure 2F, the conjunctiva samples give very high values, indicating a larger amount of mRNA, and correspond to the curves (D1) (conjunctiva of the left eye) and (F1) (conjunctiva of the right eye) [lanes 3 and 5, respectively, in the agarose gel (Figure 2F)]. Likewise, the LSCD in both eyes is confirmed by means of RT-qPCR due to the amplification of a specific 103 bp fragment of the mRNA of the *MUC5AC* gene (Figure 2F).

### CASE 5: Sample from patient 076

Patient clinically diagnosed with LSCD. The impression cytology and PAS-hematoxylin staining confirm the presence of goblet cells in the cornea (Figure 3A). Figure 3B shows the values around zero (non-amplification) and the positive values above 2,000 RFU. Likewise, the LSCD is confirmed by means of RT-qPCR due to the amplification of the specific 103 bp fragment of the mRNA of the *MUC5AC* gene (Figure 3C).

### CASE 6: Sample from patient 0121

Patient clinically diagnosed with LSCD. However, the impression cytology and PAS-hematoxylin staining do not confirm the presence of goblet cells in the cornea (Figure 4A). Figure 4B shows that the cornea sample from the patient (PTE-C) has very low values, around zero, which are correlated with those obtained by means of agarose gel electrophoresis (Figure 4C). Likewise, the samples analyzed by RT-qPCR do not show the amplification of the specific 103 bp fragment of the mRNA of the *MUC5AC* gene (Figure 4C) in the analyzed cornea.

### CASE 7: Sample from patient 0123

Patient clinically diagnosed with LSCD. The impression cytology and PAS-hematoxylin staining confirm the presence of goblet cells in the cornea (Figure 5A). Likewise, LSCD is confirmed by means of RT-qPCR due to the amplification of the specific 103 bp fragment of the mRNA of the *MUC5AC* gene (Figure 5C). The PTE-C curve (Figure 5B) corresponds to the cornea sample from the patient which in the gel corresponds to lane 4 (Figure 5C). The intensity of the band is correlated with the starting amount of mRNA.

### CASE 8: Sample from patient 0125

The impression cytology and PAS-hematoxylin staining do not show the presence of goblet cells in the cornea (Figure 6A); nevertheless, the amplification by means of RT-qPCR of the specific 103 bp fragment of the mRNA of the *MUC5AC* gene in the analyzed cornea (Figure 6C) confirms the LSCD. The PTE-C curve (Figure 6B) corresponds to the cornea sample from the patient which in the gel corresponds to lane 5 (Figure 6C) and corroborates the results obtained by agarose gel electrophoresis.

### CASE 9: Sample from patient 0132

The impression cytology and PAS-hematoxylin staining do not show the presence of goblet cells in the cornea (Figure 7A); nevertheless, the amplification by means of RT-qPCR of the specific 103 bp fragment of the mRNA of the *MUC5AC* gene in the analyzed cornea (Figure 7C) confirms the LSCD. The PTE-C curve (Figure 7B) corresponds to the cornea sample from the patient which in the gel corresponds to lane 3 (Figure 7C) and corroborates the results obtained by agarose gel electrophoresis.

### CASES 10 and 11: Samples from patients 0172 and 0186

The impression cytologies and PAS-hematoxylin staining do not show the presence of goblet cells in the corneas of any of patients 0172 and 0186 (Figures 8A and 8B); however, the amplification by means of RT-qPCR of the 103 bp fragment of the mRNA of the *MUC5AC* gene in the cornea of patient 0186 (Figure 8D) confirms the LSCD in said patient. The amplification of said 103 bp fragment of the mRNA of *MUC5AC* in the cornea of patient 0172 is not observed (Figure 8D), therefore a diagnosis of LSCD cannot be confirmed by means of RT-qPCR. Figure 8C shows curves of conjunctiva samples from both patients (0186 and 0172) with values above 2,000 RFU (positive samples). The cornea sample from patient 0172 gives values around zero and the cornea sample from patient 0186 (PTE-C 0186) gives low intensities, due to the small amount of mRNA.

### CASE 12: Sample from patient 0214

The impression cytology and PAS-hematoxylin staining do not show the presence of goblet cells in the cornea (Figure 9A); nevertheless, the amplification by means of RT-qPCR of the specific 103 bp fragment of the mRNA of the *MUC5AC* gene in the analyzed cornea (Figure 9C) confirms the LSCD. In Figure 9B, the curve corresponding to the cornea sample from the patient (PCT-C) shows values above 3,000 RFU, which indicates a very large amount of mRNA. This result is corroborated with the intensity of the band in the agarose gel.

### CASE 13: Sample from patient 0233

Patient clinically diagnosed with LSCD. The impression cytology and PAS-hematoxylin staining confirm the presence of goblet cells in the cornea (Figure 10A). Likewise, the LSCD is confirmed by means of RT-qPCR due to the amplification of the specific 103 bp fragment of the mRNA of the *MUC5AC* gene (Figure 10C). Figure 10B shows the two curves corresponding to the conjunctiva (PTE-CJ) and cornea (PTE-C) samples from the patient with values greater than 2,500 RFU.

By way of a summary, the results obtained in cases 1-13 are shown in Table 4.

**Table 4**

| Summary of the results obtained [Cases 1-13] | | | | |
|---|---|---|---|---|
| PATIENTS (code) | SIGNS | PAS-HEMATOXYLIN | PCR | DIAGNOSIS |
| 5 | + | No sample | + | + |
| 6 | + | No sample | - | - |
| 7 | + | No sample | - | - |
| 0216 (RE) | + | + | + | + |
| 0216 (LE) | + | + | + | + |
| 076 | + | + | + | + |
| 0121 | + | - | - | - |
| 0123 | + | + | + | + |
| 0125 | + | - | + | + |
| 0132 | + | - | + | + |
| 0172 | + | - | - | - |
| 0186 | + | - | + | + |
| 0214 | + | - | + | + |
| 0233 | + | + | + | + |

### Conclusions

As can be seen, the correlation is greater between the clinical diagnosis and the impression cytology analyzed by RT-PCR than between the clinical diagnosis and the impression cytology stained with PAS-hematoxylin. The sensitivity and the specificity of the described technique (RT-PCR) are greater than those of the conventional impression cytology stained with PAS-hematoxylin.

There are 4 manifestations of LSCD which are clinically but not molecularly diagnosed. In these cases the medical record of the studied patients was reviewed, suspecting an erroneous clinical diagnosis.

By means of impression cytology stained with PAS-hematoxylin, LSCD could only be diagnosed in 5 cases of the 9 cases analyzed by the two methods and which were positive [patients 0216 (2 eyes), 076, 0123 and 0244]. There are 4 cases which, analyzed by means of impression cytology stained with PAS-hematoxylin, can be considered as "false negatives" since they were negative by means of that technique but positive by means of RT-qPCR, resulting in an erroneous diagnosis [patients 0125, 032, 0186 and 0214].

In view of these results, the success of the diagnosis of LSCD by PCR (sensitivity of the technique), can be estimated at 100% in the analyzed cases, compared to 45% of the technique based on PAS-hematoxylin staining.

## Claims

1. An *in vitro* method for diagnosing limbal stem cell deficiency (LSCD) in a subject, comprising detecting the expression of the *MUC5AC* gene in a cornea sample from said subject, wherein the detection of expression of the *MUC5AC* gene in said cornea sample is indicative of the subject suffering from LSCD.

2. An *in vitro* method for diagnosing limbal stem cell deficiency (LSCD) in a subject, comprising:
a) quantifying the expression of the *MUC5AC* gene in a cornea sample from said subject; and
b) comparing the expression level of the *MUC5AC* gene detected in step (a) with the expression level of the *MUC5AC* gene in a reference sample;
wherein an increase in the expression level of the *MUC5AC* gene detected in step (a) with respect to the expression level of the *MUC5AC* gene in the reference sample is indicative of LSCD.

3. The method according to claim 2, wherein step a) comprises quantifying the expression of the *MUC5AC* gene by means of RT-qPCR in a cornea sample from said subject, using a pair of oligonucleotide primers formed by an oligonucleotide primer comprising the nucleotide sequence identified as SEQ ID NO: 1 (MUC5AC-RT-F2) and by an oligonucleotide primer comprising the nucleotide sequence identified as SEQ ID NO: 2 (MUC5AC-RT-R2), under conditions which allow amplifying a specific fragment of the *MUC5AC* gene.

4. The method according to any of claims 2 or 3, wherein said reference sample is a cornea sample from said subject.

5. An oligonucleotide selected from an oligonucleotide comprising the nucleotide sequence shown in SEQ ID NO: 1, an oligonucleotide comprising the nucleotide sequence shown in SEQ ID NO: 2 and combinations of both oligonucleotides.

6. A kit comprising at least one oligonucleotide according to claim 5, or a pair of oligonucleotide primers formed by an oligonucleotide primer comprising the nucleotide sequence shown in SEQ ID NO: 1 and by an oligonucleotide primer comprising the nucleotide sequence shown in SEQ ID NO: 2.

7. The kit according to claim 6, further comprising a substance labeled with a fluorophore.

8. The kit according to claim 7, wherein said substance labeled with a fluorophore comprises a specific Taqman type probe suitable for identifying the *MUC5AC* gene.

9. Use of a kit according to any of claims 6 to 8 in a method for diagnosing limbal stem cell deficiency in a subject.

10. Use of a pair of oligonucleotides formed by an oligonucleotide comprising the nucleotide sequence shown in SEQ ID NO: 1 and an oligonucleotide comprising the nucleotide sequence shown in SEQ ID NO: 2, as oligonucleotide primers in a PCR for detecting and identifying the *MUC5AC* gene, or for quantifying said *MUC5AC* gene, or in a method for the diagnosis of LSCD by means of detecting and/or quantifying the *MUC5AC* gene.

11. A method for evaluating if a patient with a corneal pathology susceptible to keratoplasty is a suitable candidate for keratoplasty, comprising:
- detecting the expression of the *MUC5AC* gene in a cornea sample from said patient; or, alternatively
- determining the expression level of the *MUC5AC* gene in a cornea sample from said patient; and
if the *MUC5AC* gene is not detected in said cornea sample from said patient; or, alternatively, if the expression level of the *MUC5AC* gene detected in the cornea sample from said patient is equal to the expression level of the *MUC5AC* gene in a reference sample, then said patient is a suitable candidate for corneal transplantation or keratoplasty.

12. A method for evaluating if a patient with a corneal pathology susceptible to keratoplasty is a suitable candidate for keratoplasty, comprising subjecting said patient to an assay for diagnosing if the patient suffers from limbal stem cell deficiency (LSCD), and, if said patient does not suffer from LSCD, then said patient is a suitable candidate for keratoplasty.

13. The method according to claim 12, wherein said assay for diagnosing if the subject suffers from LSCD is carried out by means of a method such as the one defined in any of claims 1 to 5.
